# EUROPEAN PATENT APPLICATION

(11) **EP 2 070 525 A1**
(43) Date of publication of application: **17.06.2009**
(21) Application number: 08171093.1
(22) Date of filing: 09.12.2008
(51) Int. Cl.: A61K 31/05, A61K 36/575, A61P 29/02, A61P 19/02

(54) **Compositions comprising Magnolol and/or Honokiol and chondroitin and use thereof for the treatment, co-treatment or prevention of inflammatory disorders**

(30) Priority: 11.12.2007 EP 07023948; 11.12.2007 EP 07023985
(71) Applicant: DSM IP Assets B.V., 6411 TE Heerlen (NL)
(72) Inventor: Raederstorff, Daniel, 68720, Flaxlanden (FR); Richard, Nathalie, 68100, Mulhouse (FR); Schwager, Joseph, 4056, Basel (CH); Wertz, Karin, 79618, Rheinfelden (FR)
(74) Representative: Schwander, Kuno

(57) **Abstract**

The present invention relates to novel compositions comprising magnolol and/ or honokiol and chondroitin as well as to the use of these compositions as a medicament, in particular as a medicament for the treatment, co-treatment or prevention of inflammatory disorders.

## Description

The present invention relates to novel compositions comprising magnolol and/ or honokiol and chondroitin as well as to the use of these compositions as a medicament, in particular as a medicament for the treatment, co-treatment or prevention of inflammatory disorders.

Inflammatory disorders are one of the most important health problems in the world. Inflammation is in general a localized protective response of the body tissues to invasion of the host by foreign material or injurious stimuli. The causes of inflammation can be infectious agents such as bacteria, viruses, and parasites; or physical agents such as burns or radiation; or chemicals like toxins, drugs or industrial agents; or immunological reactions such as allergies and autoimmune responses or conditions associated with oxidative stress.

Inflammation is characterized by pain, redness, swelling, heat, and eventual loss of function of the affected area. These symptoms are the results of a complex series of interactions mainly taking place between the cells of the immune system. The response of the cells results in an interacting network of several groups of inflammatory mediators: Proteins (*e*.*g*.,cytokines, enzymes [*e*.*g*.,proteases, peroxydase], major basic protein, adhesion molecules [ICAM, VCAM]), lipid mediators (*e*.*g*.,eicosanoids, prostaglandins, leukotrienes, platelet activating factor [PAF]), reactive oxygen species (*e*.*g*.,hydroperoxides, superoxyde anion O₂⁻, nitric oxide [NO] etc). However, many of those mediators of inflammation are also regulators of normal cellular activity. Thus, deficiencies of inflammatory reactions lead to a compromised host (*i*.*e*. infection) while uncontrolled and thus chronic inflammation leads to inflammatory diseases mediated in part by the excessive production of several of the above mentioned mediators.

Acute and chronic inflammation resulting from an excessive biosynthesis of inflammatory mediators is involved in numerous inflammatory disorders such as arthritis (*e*.*g*. osteoarthritis, rheumatoid arthritis), asthma, inflammatory bowel diseases, inflammatory diseases of the skin (*e*.*g*. contact dermatitis [particularly diaper area dermatitis], atopic dermatitis, xerosis, eczema, rosacea, seborrhea, psoriasis, neurodermitis, acne, thermal and radiation burns such as sunburn,) and chronic inflammatory disorders, such as atherosclerosis, heart diseases, metabolic syndrome X, cancer, Alzheimer's disease and pre-stages thereof such as mild cognitive impairment or photoageing which is associated with chronic skin inflammation.

Rheumatoid arthritis is a chronic inflammatory disease of the joints and is one of many different forms of arthritis. For example, arthritis includes rheumatoid arthritis, spondyloarthopathies, gouty arthritis, osteoarthritis, systemic lupus erythematosus and juvenile arthritis. Like asthma, rheumatoid arthritis is characterized at the molecular level by chronically unbalanced expression of cytokines, chemokines, kinins and their receptors, adhesion molecules and their respective receptors, as well as inflammatory enzymes.

Psoriasis is one of the most common skin problems, affecting 1-3% of the human population. Inflammatory bowel disease is a general term used to describe gastrointestinal tract diseases and includes disorders such as ulcerative colitis and Crohn's disease.

Beside the process of intravascular lipid deposition, inflammatory reactions of the endothelial (*i*.*e*. blood vessel) wall are considered to critically contribute to atherosclerosis *i*.*e*. atheroma formation. Atherosclerosis results from vascular injury which triggers inflammation. Activated macrophages, T-lymphocytes, and eventually smooth muscle cells are present in atherosclerotic plaques. Monocyte/macrophage and lymphocyte activation leads to the release of eicosanoids, cytokines and matrix metalloproteinases (MMPs) which are implicated in endothelial damage, as well as in the formation and eventually the rupture of atherosclerotic plaques. Finally, circulating inflammatory markers such as C-reactive protein (CRP), fibrinogen, and interleukins are increased or altered in groups at high-risk of coronary artery diseases (CAD). Several clinical trials indicate that elevated CRP concentration correlates with increased risk of coronary, and vascular, events. Thus inflammation appears to play an important role in the initiation and progression of atheroma formation.

Inflammatory processes are also associated with the pathophysiology of Alzheimer's disease. There is evidence of inflammation in the brain of patients with Alzheimer's disease, as it is characterized by increased levels of cytokines and activated microglial cells. Thus, inflammation is not only involved in the classical inflammatory disorders (*e*.*g*., arthritis, asthma, bowel diseases) but is also associated with many chronic inflammatory disorders (*e*.*g*., atherosclerosis, heart diseases, metabolic syndrome X, cancer, Alzheimer disease).

Inflammatory events are also associated with the pathophysiology of different types of cancers (*e*.*g*. gastric and intestinal cancers, melanomas). Increased levels of inflammatory mediators such as prostaglandins have been found in cancers of breast, colon, lung and pancreas in humans.

Currently, two main classes of drugs, the corticosteroid and the nonsteroidal anti-inflammatory drugs (NSAIDs) are used to treat inflammatory disorders. NSAIDs and corticosteroids provide essentially symptomatic relief. Use of corticosteroids has declined due to a growing concern about the serious side effects of prolonged use.

NSAIDs are among the most widely used drugs, primarily for the treatment of pain and inflammatory disorders, in particular for the treatment of arthritis (*i*.*e*. pain relief). Epidemiological studies have suggested that patients taking NSAIDs have a lower risk of developing Alzheimer's disease than those not taking NSAIDs. A protective effect of NSAIDs suggests that the cyclooxygenases might be involved in the neurodegenerative process. Epidemiological studies showed a significant reduction in the risk of colorectal, gastric, esophageal, and breast cancers among people who take NSAIDs compared with those not taking NSAIDs. In animal models, NSAIDs significantly reduced tumor development.

However, long-term use of NSAIDs when treating chronic diseases such as arthritis, is limited by severe side-effects like serious gastrointestinal complications, renal toxicity or asthmatic reactions.

Therefore, there is a need for new anti-inflammatory agents with weak or no side effects. Patients with inflammatory diseases have a special interest in a type of treatment considered as "natural" with mild anti-inflammatory effects and without major side effects, which can be used for disease prevention and as adjuvant treatment. Furthermore, the treatment used needs to maintain the equilibrium between excessive and insufficient inflammatory reaction.

There are many known examples of such "natural" agents with shown anti-inflammatory action. However, a disadvantage of these "natural" compounds is that their biological and thus inhibitory activity is often inadequate. When two or more natural substances are applied concomitantly, their action can be additively or even synergistically enhanced. This lowers the required amount of each substance in order to effect disease development or treatment. Since lower doses of each of the natural substances individually can be used, there is less chance that deleterious levels are reached and also less chance of serious side-effects due to chronic use.

Surprisingly, it has been found that a combination of magnolol and/ or honokiol and chondroitin synergistically enhances the anti-inflammatory activity. Furthermore, it has surprisingly been found, that this combination also synergistically enhances cartilage build-up and repair by stimulating the proliferation of chondrocytes. Therefore, the composition of the present invention may be especially useful in the treatment, co-treatment and prevention of inflammatory disorders, such as heart disease, multiple sclerosis, osteo- and rheumatoid arthritis, atherosclerosis, and osteoporosis.

Thus, the invention relates to a composition comprising magnolol and/ or honokiol and chondroitin. Preferably, the invention relates to a composition comprising magnolol, honokiol and chondroitin.

The ratio of magnolol and/ or honokiol to chondroitin in the compositions according to the invention may be selected in the range of 1 to 50 to 50 to 1, preferably in the range of 1 to 25 to 25 to 1, such as e.g.about 1 to 10 to 1 to 1.

The composition of the present invention is especially suitable for the treatment, co-treatment and prevention of different forms of arthritis, in particular osteoarthritis and rheumatoid arthritis. Also, the compositions of the present invention are suitable as an agent for treatment, co-treatment and prevention of joint disorders in particular for reduction of joint inflammation, maintenance and/or increase of joint health, prevention of joint stiffness, increase of joint mobility, providing supple and/or flexible joints, lubrication of the joints, relief of pain associated with joint inflammation, decrease of joint swelling, lessening joint problems, and providing joint care. Thus, the invention also relates to the use of a composition according to the invention as an agent for the treatment, co-treatment or prevention of inflammatory disorders as well as joint disorders.

In another aspect the invention relates to the use of a composition of the invention as an agent for the treatment, co-treatment or prevention of inflammatory disorders more preferably of arthritis or skin inflammation, most preferably of osteoarthritis or sunburn.

In a different aspect, the invention also relates to the composition of the invention for use as a medicament.

In yet another embodiment, the invention relates to the use of a composition according to the invention for the manufacture of a nutraceutical, pharmaceutical, cosmetic or dermatological preparation suitable for the treatment, co-treatment or prevention of inflammatory disorders, more preferably of arthritis, in particular of osteoarthritis. Furthermore, the invention relates to the use of a composition according to the invention for the manufacture of a nutraceutical, pharmaceutical, cosmetic or dermatological preparation suitable for the treatment, co-treatment or prevention of inflammatory disorders such as skin inflammation, in particular of sunburn.

Also, the invention relates to a method for treatment, co-treatment and prevention of inflammatory disorders, in particular of arthritis, more in particular of osteoarthritis or rheumatoid arthritis, in animals including humans said method comprising the step of administering 'an effective amount of the composition according to the invention' to animals including humans, which are in need thereof. Preferably, the inflammatory disorder is arthritis, most preferably osteoarthritis.

The term 'an effective amount of the composition according to the invention' refers to an amount necessary to obtain a physiological effect. The physiological effect may be achieved by one single dose or by repeated doses. The dosage administered may, of course, vary depending upon known factors, such as the physiological characteristics of the particular composition and its mode and route of administration; the age, health and weight of the recipient; the nature and extent of the symptoms; the kind of concurrent treatment; the frequency of treatment; and the effect desired and can be adjusted by a person skilled in the art.

In the framework of the invention, with animals is meant all animals, including mammals, examples of which include humans. Preferred examples of mammals beside humans are non-ruminant or ruminant animals including cats, dogs, dromedaries, camels, elephants, and horses.

In another embodiment the invention relates to a nutraceutical composition comprising the composition according to the invention and a nutraceutically acceptable carrier.

The term nutraceutical composition as used herein include food product, foodstuff, dietary supplement, nutritional supplement or a supplement composition for a food product or a foodstuff.

Thus, in another embodiment the present invention relates to a nutraceutical wherein the nutraceutical is a food product, foodstuff, dietary supplement, nutritional supplement or a supplement composition for a food product or a foodstuff.

As used herein, the term food product refers to any food or feed suitable for consumption by humans or animals. The food product may be a prepared and packaged food (*e*.*g*., mayonnaise, salad dressing, bread, or cheese food) or an animal feed (*e*.*g*., extruded and pelleted animal feed, coarse mixed feed or pet food composition). As used herein, the term foodstuff refers to any substance fit for human or animal consumption. The term dietary supplement refers to a small amount of a compound for supplementation of a human or animal diet packaged in single or multiple dose units. Dietary supplements do not generally provide significant amounts of calories but may contain other micronutrients (*e*.*g*., vitamins or minerals). The term nutritional supplement refers to a composition comprising a dietary supplement in combination with a source of calories. In some embodiments, nutritional supplements are meal replacements or supplements (*e*.*g*., nutrient or energy bars or nutrient beverages or concentrates).

Food products or foodstuffs are for example beverages such as non-alcoholic and alcoholic drinks as well as liquid preparation to be added to drinking water and liquid food, non-alcoholic drinks are for instance soft drinks, sport drinks, fruit juices, such as for example orange juice, apple juice and grapefruit juice; lemonades, teas, near-water drinks and milk and other dairy drinks such as for example yoghurt drinks, and diet drinks. In another embodiment food products or foodstuffs refer to solid or semi-solid foods comprising the composition according to the invention. These forms can include, but are not limited to baked goods such as cakes and cookies, puddings, dairy products, confections, snack foods, or frozen confections or novelties (*e*.*g*., ice cream, milk shakes), prepared frozen meals, candy, snack products (*e*.*g*., chips), liquid food such as soups, spreads, sauces, salad dressings, prepared meat products, cheese, yogurt and any other fat or oil containing foods, and food ingredients (*e*.*g*., wheat flour). The term food products or foodstuffs also includes functional foods and prepared food products, the latter referring to any pre-packaged food approved for human consumption.

Animal feed including pet food compositions advantageously include food intended to supply necessary dietary requirements, as well as treats (*e*.*g*., dog biscuits) or other food supplements. The animal feed comprising the composition according to the invention may be in the form of a dry composition (for example, kibble), semi-moist composition, wet composition, or any mixture thereof. Alternatively or additionally, the animal feed is a supplement, such as a gravy, drinking water, yogurt, powder, suspension, chew, treat (*e*.*g*., biscuits) or any other delivery form.

Dietary supplements of the present invention may be delivered in any suitable format. In preferred embodiments, dietary supplements are formulated for oral delivery. The ingredients of the dietary supplement of this invention are contained in acceptable excipients and/or carriers for oral consumption. The actual form of the carrier, and thus, the dietary supplement itself, is not critical. The carrier may be a liquid, gel, gelcap, capsule, powder, solid tablet (coated or non-coated), tea, or the like. The dietary supplement is preferably in the form of a tablet or capsule and most preferably in the form of a hard (shell) gelatin capsule. Suitable excipient and/or carriers include maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and the like (including mixtures thereof). Preferred carriers include calcium carbonate, magnesium stearate, maltodextrin, and mixtures thereof. The various ingredients and the excipient and/or carrier are mixed and formed into the desired form using conventional techniques. The tablet or capsule of the present invention may be coated with an enteric coating that dissolves at a pH of about 6.0 to 7.0. A suitable enteric coating that dissolves in the small intestine but not in the stomach is cellulose acetate phthalate. Further details on techniques for formulation for and administration may be found in the latest edition of Remington's Pharmaceutical Sciences (Maack Publishing Co., Easton, PA).

In other embodiments, the dietary supplement is provided as a powder or liquid suitable for adding by the consumer to a food or beverage. For example, in some embodiments, the dietary supplement can be administered to an individual in the form of a powder, for instance to be used by mixing into a beverage, or by stirring into a semi-solid food such as a pudding, topping, sauce, puree, cooked cereal, or salad dressing, for instance, or by otherwise adding to a food e.g. enclosed in caps of food or beverage container for release immediately before consumption. The dietary supplement may comprise one or more inert ingredients, especially if it is desirable to limit the number of calories added to the diet by the dietary supplement. For example, the dietary supplement of the present invention may also contain optional ingredients including, for example, herbs, vitamins, minerals, enhancers, colorants, sweeteners, flavorants, inert ingredients, and the like.
In some embodiments, the dietary supplements further comprise vitamins and minerals including, but not limited to, calcium phosphate or acetate, tribasic; potassium phosphate, dibasic; magnesium sulfate or oxide; salt (sodium chloride); potassium chloride or acetate; ascorbic acid; ferric orthophosphate; niacinamide; zinc sulfate or oxide; calcium pantothenate; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D3; cyanocobalamin; sodium selenite; copper sulfate; vitamin A; vitamin C; inositol; potassium iodide. Suitable dosages for vitamins and minerals may be obtained, for example, by consulting the U.S. RDA guidelines.

In other embodiments, the present invention provides nutritional supplements (*e*.*g*., energy bars or meal replacement bars or beverages) comprising the composition according to the invention. The nutritional supplement may serve as meal or snack replacement and generally provide nutrient calories. Preferably, the nutritional supplements provide carbohydrates, proteins, and fats in balanced amounts. The nutritional supplement can further comprise carbohydrate, simple, medium chain length, or polysaccharides, or a combination thereof. A simple sugar can be chosen for desirable organoleptic properties. Uncooked cornstarch is one example of a complex carbohydrate. If it is desired that it should maintain its high molecular weight structure, it should be included only in food formulations or portions thereof which are not cooked or heat processed since the heat will break down the complex carbohydrate into simple carbohydrates, wherein simple carbohydrates are mono- or disaccharides. The nutritional supplement contains, in one embodiment, combinations of sources of carbohydrate of three levels of chain length (simple, medium and complex; *e*.*g*., sucrose, maltodextrins, and uncooked cornstarch).
Sources of protein to be incorporated into the nutritional supplement of the invention can be any suitable protein utilized in nutritional formulations and can include whey protein, whey protein concentrate, whey powder, egg, soy flour, soy milk soy protein, soy protein isolate, caseinate (*e*.*g*., sodium caseinate, sodium calcium caseinate, calcium caseinate, potassium caseinate), animal and vegetable protein and hydrolysates or mixtures thereof. When choosing a protein source, the biological value of the protein should be considered first, with the highest biological values being found in caseinate, whey, lactalbumin, egg albumin and whole egg proteins. In a preferred embodiment, the protein is a combination of whey protein concentrate and calcium caseinate. These proteins have high biological value; that is, they have a high proportion of the essential amino acids. See Modern Nutrition in Health and Disease, eighth edition, Lea & Febiger, publishers, 1986, especially Volume 1, pages 30-32. The nutritional supplement can also contain other ingredients, such as one or a combination of other vitamins, minerals, antioxidants, fiber and other dietary supplements (*e*.*g*., protein, amino acids, choline, lecithin, omega-3 fatty acids). Selection of one or several of these ingredients is a matter of formulation, design, consumer preference and end-user. The amounts of these ingredients added to the dietary supplements of this invention are readily known to the skilled artisan. Guidance to such amounts can be provided by the U.S. RDA doses for children and adults. Further vitamins and minerals that can be added include, but are not limited to, calcium phosphate or acetate, tribasic; potassium phosphate, dibasic; magnesium sulfate or oxide; salt (sodium chloride); potassium chloride or acetate; ascorbic acid; ferric orthophosphate; niacinamide; zinc sulfate or oxide; calcium pantothenate; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D3 ; cyanocobalamin; sodium selenite; copper sulfate; vitamin A; vitamin C; inositol; potassium iodide.

The nutritional supplement can be provided in a variety of forms, and by a variety of production methods. In a preferred embodiment, to manufacture a food bar, the liquid ingredients are cooked; the dry ingredients are added with the liquid ingredients in a mixer and mixed until the dough phase is reached; the dough is put into an extruder, and extruded; the extruded dough is cut into appropriate lengths; and the product is cooled. The bars may contain other nutrients and fillers to enhance taste, in addition to the ingredients specifically listed herein.
It is understood by those of skill in the art that other ingredients can be added to those described herein, for example, fillers, emulsifiers, preservatives, etc. for the processing or manufacture of a nutritional supplement.

Additionally, flavors, coloring agents, spices, nuts and the like may be incorporated into the nutraceutical composition. Flavorings can be in the form of flavored extracts, volatile oils, chocolate flavorings, peanut butter flavoring, cookie crumbs, crisp rice, vanilla or any commercially available flavoring. Examples of useful flavoring include, but are not limited to, pure anise extract, imitation banana extract, imitation cherry extract, chocolate extract, pure lemon extract, pure orange extract, pure peppermint extract, imitation pineapple extract, imitation rum extract, imitation strawberry extract, or pure vanilla extract; or volatile oils, such as balm oil, bay oil, bergamot oil, cedarwood oil, walnut oil, cherry oil, cinnamon oil, clove oil, or peppermint oil; peanut butter, chocolate flavoring, vanilla cookie crumb, butterscotch or toffee. In one embodiment, the dietary supplement contains cocoa or chocolate.

Emulsifiers may be added for stability of the nutraceutical compositions. Examples of suitable emulsifiers include, but are not limited to, lecithin (*e*.*g*., from egg or soy), and/or mono- and diglycerides. Other emulsifiers are readily apparent to the skilled artisan and selection of suitable emulsifier(s) will depend, in part, upon the formulation and final product. Preservatives may also be added to the nutritional supplement to extend product shelf life. Preferably, preservatives such as potassium sorbate, sodium sorbate, potassium benzoate, sodium benzoate or calcium disodium EDTA are used.
In addition to the carbohydrates described above, the nutraceutical composition can contain natural or artificial (preferably low calorie) sweeteners, *e*.*g*., saccharides, cyclamates, aspartamine, aspartame, acesulfame K, and/or sorbitol. Such artificial sweeteners can be desirable if the nutritional supplement is intended to be consumed by an overweight or obese individual, or an individual with type II diabetes who is prone to hyperglycemia.
Moreover, a multi-vitamin and mineral supplement may be added to the nutraceutical compositions of the present invention to obtain an adequate amount of an essential nutrient, which is missing in some diets. The multi-vitamin and mineral supplement may also be useful for disease prevention and protection against nutritional losses and deficiencies due to lifestyle patterns.

The dosage and ratios of magnolol and/ or honokiol and chondroitin administered via a nutraceutical will, of course, vary depending upon known factors, such as the physiological characteristics of the particular composition and its mode and route of administration; the age, health and weight of the recipient; the nature and extent of the symptoms; the kind of concurrent treatment; the frequency of treatment; and the effect desired which can be determined by the expert in the field with normal trials, or with the usual considerations regarding the formulation of a nutraceutical composition.

In a preferred embodiment, the nutraceutical comprises per serving an amount of 1 mg to 1000mg, more preferably 2 mg to 500 mg of magnolol and/ or honokiol preferably in the form of a magnolia bark extract comprising both magnolol and honokiol and 1 mg to 2000 mg, preferably 5 mg to 1000 mg of chondroitin.

In another aspect, the invention relates to a pharmaceutical comprising the composition according to the invention and a pharmaceutically acceptable carrier.

A person skilled in the art knows which carriers can be used as pharmaceutically acceptable carriers. Suitable pharmaceutical carriers are *e*.*g*. described in Remington's Pharmaceutical Sciences, supra, a standard reference text in this field. Examples of such pharmaceutically acceptable carriers are both inorganic and organic carrier materials, suitable for oral/parenteral/injectable administration and include water, gelatin, gum arabic, lactose, starch, magnesium stearate, talc, vegetable oils, and the like.

The pharmaceutical composition may further comprise conventional pharmaceutical additives and adjuvants, excipients or diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers, and the like.

In a preferred embodiment the pharmaceutical is in the form of a powder, tablet, capsule, gel, liquid or solid embodiment.

The dosages and ratios of the individual components in a pharmaceutical composition can be determined by the expert in the field with normal preclinical and clinical trials, or with the usual considerations regarding the formulation of pharmaceutical composition.

In a preferred embodiment the active ingredients are administered via a pharmaceutical composition either in the form of a single dose or by multiple doses in an amount of at least 0.01 mg/ kg bodyweight/ day, preferably of 0.1-50 mg/ kg body weight/ day, most preferably of 0.3-15 mg/ kg body weight / day such as e.g. 0.8 to 8 mg/ kg body weight / day of magnolol and/ or honokiol and in an amount of at least 0.01 mg/ kg bodyweight/ day preferably 0.1-50 mg/ kg body weight/ day, most preferably 0.3-15 mg/ kg body weight / day such as 3-25 mg/ kg body weight / day of chondroitin.

A pharmaceutical may for example comprise magnolol and/ or honokiol in an amount from 1 mg to 500 mg and chondroitin in an amount of 10 mg to 1000 mg per dosage unit, *e*.*g*., per capsule or tablet, or from 1 mg to 500 mg of magnolol and/ or honokiol and from 500 mg to 2000 mg of chondroitin in a liquid formulation

The nutraceutical and pharmaceutical according to the present invention may be in any galenic form that is suitable for administering to the animal body including the human body, more in particular in any form that is conventional for oral administration, *e*.*g*. in solid form, for example as (additives/supplements for) food or feed, food or feed premixes, fortified food or feed, tablets, pills, granules, dragées, capsules, and effervescent formulations such as powders and tablets, or in liquid form, for instance in the form of solutions, emulsions or suspensions, for example as beverages, pastes and oily suspensions. The pastes may be filled into hard or soft shell capsules. Examples for other application forms are forms for transdermal, parenteral, topical or injectable administration. The nutraceutical and pharmaceutical may be in the form of controlled (delayed) release formulation. Examples of pharmaceuticals also include compositions suitable for topical application such as crèmes, gels, sprays, dry sticks, powders etc.

The term "magnolol" as used herein comprises the pure compound also known as 5,5'-Diallyl-2,2'-biphenyldiol or 5,5'-di-2-propenyl-[1,1'-Biphenyl]-2,2'-diol (CAS [528-43-8]) and plant extracts containing the same. The term magnolol also comprises etherified or esterified hydroxy derivatives from 5,5'-Diallyl-2,2'-biphenyldiol or 5,5'-di-2-propenyl-[1,1'-Biphenyl]-2,2'-diol. The ester or ether groups may for example be derived from straight or branched alkyl groups having 1 to 26 carbon atoms or from substituted or unsubstituted straight or branched aliphatic, araliphatic or aromatic carboxylic acids having 1 to 26 carbon atoms. Examples of etherified hydroxy groups further include glycoside groups. Examples of esterified hydroxy group further include glucuronide or sulfate groups. Preferably magnolol as used herein is 5,5'-Diallyl-2,2'-biphenyldiol or 5,5'-di-2-propenyl-[1,1'-Biphenyl]-2,2'-diol.

The term "honokiol" as used herein comprises the pure compound also known as 3',5-Diallyl-2,4'-biphenyldiol or 3',5-di-2-propenyl-[1,1'-Biphenyl]-2,4'-diol (CAS [35354-74-6]) and plant extracts containing the same.
The term honokiol also comprises etherified or esterified hydroxy derivatives from 3',5-Diallyl-2,4'-biphenyldiol or 3',5-di-2-propenyl-[1,1'-Biphenyl]-2,4'-diol. The ester or ether groups may for example be derived from straight or branched alkyl groups having 1 to 26 carbon atoms or from substituted or unsubstituted straight or branched aliphatic, araliphatic or aromatic carboxylic acids having 1 to 26 carbon atoms. Examples of etherified hydroxy groups further include glycoside groups. Examples of esterified hydroxy group further include glucuronide or sulfate groups. Preferably, "honokiol" as used herein is 3',5-Diallyl-2,4'-biphenyldiol or 3',5-di-2-propenyl-[1,1'-Biphenyl]-2,4'-diol.

Magnolol and/ or honokiol may be obtained either by chemical synthesis or by isolation from plant material or by mixtures thereof. Alternatively, magnolol and/ or honokiol may be used in the form of a composition comprising the magnolol and/ or honokiol such as a plant extract. Thus, the term magnolol and/ or honokiol encompasses both "natural" (isolated) and "synthetic" (manufactured). The term magnolol and/ or honokiol also includes any plant material or extract comprising at least one of magnolol or honokiol. The amount of magnolol and/ or honokiol in a plant material or extract is not critical. Preferably an amount of at least 1 wt.% of at least one of magnolol or honokiol based on the total weight of the plant material or extract is present. More preferably an amount of at least 50 wt.%, even more preferably an amount of at least 90 wt. % based on the total weight of the plant material or extract is present.

Preferably, magnolol and/ or honokiol are used in the form of a plant extract derived from the bark of *Magholia officinalis* obtainable *e*.*g*. by stripping the bark from stems, branches, and roots. If desirable, the plant extract comprising the biphenyl derivatives may be prepared, for example, by processing a natural source of Magnolia bark such that at least one of magnolol or honokiol has been selectively removed, retained, or enriched. Alternatively, purified magnolol and/ or honokiol can be used to make such compositions. Such a composition can also be prepared, for example, by adding an amount of at least one of magnolol and/ or honokiol to a natural source or processed form of a natural source comprising the magnolol and/ or honokiol.

Plant extracts containing the magnolol and/ or honokiol include extracts from *Magnolia officinalis*, *Magnolia obovata, Magnolia rostrata, Magnolia bilboa, Magnolia biondii, Magnolia quinquepeta, Magnolia sprengeri, Manglietia insignis, Manglietia szechuanica, Hanglietia yuyuanensis*, *Cercidiphyllum japonicum*, *Machilus thunbergii* and others.

Magnolol and/ or honokiol are preferably derived from *Magnolia bark* that may be obtained from conventional and commercially available sources. The *Magnolia bark* derived magnolol and/ or honokiol are obtainable via a number of methods known in the art.

The magnolia bark may be processed by any suitable means to obtain magnolol and/ or honokiol'. Plant extracts containing at least one of magnolol and/ or honokiol may be obtained by conventional extraction of Magnolia bark (optionally in dried or grinded form) with solvents like ethanol, dichloromethane at reflux temperature or at lower temperature. Alternatively, it may be extracted with supercritical fluids like liquid carbon dioxide or by steam distillation of the bark with water followed by sampling of the distilled organic part. Sampling may for example be done by extraction with an organic solvent like dichloromethane. Subsequent removal of the solvent gives the desired magnolia bark extract. Optionally, the thus obtained magnolia bark extract may be subjected to further processing steps to enrich the content of magnolol and/ or honokiol. Extraction and/ or purification processes are *e*.*g*. described in Journal of Separation Science (2006), 29(3), 351-357 or Journal of Chromatography, A (2004), 1036(2), 171-175 without being limited thereto. If desirable, the extract or the pure isolated compounds may be further derivatized by methods known by a person skilled in the art *e*.*g*. using alkylating or acetylating agents in order to obtain the desired derivatives.

More preferably, the plant extract comprising magnolol and/ or honokiol is obtained by supercritical carbon dioxide extraction of Magnolia bark.

The magnolol and/ or honokiol may alternatively be obtained by chemical synthesis. Honokiol may *e*.*g*. be synthesized starting from 5-bromosalicylic acid and p-hydroxybenzoic acid by utilizing a Pd-catalyzed Suzuki-Miyaura coupling reaction as a key step as described in Bioorganic & Medicinal Chemistry Letters (2004), 14(10), 2621-2625. A synthesis of magnolol is *e*.*g*. described in JP 2004292392.

Preferably magnolol and/ or honokiol are used in the form of an extract from the bark of *Magnolia officinalis* in compositions according to the invention.

Preferably magnolol and/ or honokiol are used in the form of an extract from the bark of *Magnolia officinalis*. Preferably, an extract is used which comprises both, magnolol and honokiol. The mol ratio of magnolol and honokiol in the '*Magnolia Bark extract*' is preferably in the range of about 1.25 to 0.5, most preferably about 1.1 to 0.8 such as e.g. about 1.
If a Magnolia bark extract is used instead of the pure compounds magnolol and honokiol, the dosages and ratios given in this application can be easily adjusted by a skilled person in the art knowing the content of magnolol and honokiol in such an extract.

In the framework of the present invention, with chondroitin is meant a sulfated glycosaminoglycan (GAG) composed of repeating disaccharide subunits. The source material for chondroitin may be bovine cartilage. The chains of the disaccharides vary in length from 20 to 80. Daily intake of chondroitin by a human adult (weighing approximately 70kg) is preferably between 100 and 2000 mg, more preferably between 800 to 1200 mg per day.

In another aspect, the invention relates to a cosmetic or dermatological preparation (the latter preparation are a specific type of a pharmaceutical) comprising an effective amount of the composition of the invention and a cosmetically or dermatologically acceptable carrier.
The cosmetic or dermatological composition may further comprise conventional cosmetic respectively dermatological adjuvants and/or additives and/or additional active ingredients.

Preferably the cosmetic or dermatological preparations are skin care formulations for the treatment, co-treatment or prevention of inflammation of the skin, in particular of sunburn caused by UV-radiation, of contact dermatitis (particularly diaper area dermatitis), atopic dermatitis, xerosis, eczema, rosacea, seborrhea, psoriasis, neurodermitis, thermal burns, photoageing or for the treatment, co-treatment or prevention of impure skin. Examples of impure skin include pimples, acne and other skin impurities with an inflammatory aspect.
The term "effective amount" means preferably at least 0.001 % of magnolol and/ or honokiol and chondroitin based on the total weight of the cosmetic or dermatological composition. Preferably, the cosmetic or dermatological preparations comprise magnolol and/ or honokiol and chondroitin in a total amount of about 0.01 wt.-% and 20 wt.-%, more preferably of about 0.05 and 10 wt.-%, still more preferably of about 0.1 and 5 wt.-%.

The amount of the cosmetic or dermatological preparation which is to be applied to the skin depends on the concentration of the active ingredients in the preparation and the desired cosmetic or pharmaceutical effect. For example, the application can be such that a crème is applied to the skin. A crème is usually applied in an amount of about 1 to 2 mg crème/cm² skin. The amount of the composition which is applied to the skin is, however, not critical, and if with a certain amount of applied composition the desired effect cannot be achieved, a higher concentration of the active preparations which contain more active ingredient might be employed.

The invention also relates to the use of the cosmetic preparation for the cosmetic treatment, co-treatment or prevention of inflammation of the skin, in particular for the cosmetic treatment, co-treatment or prevention of sunburn, contact dermatitis (particularly diaper area dermatitis), atopic dermatitis, xerosis, eczema, rosacea, seborrhea, psoriasis, neurodermitis, thermal burns or photoageing.

Also, the invention relates to a method for the treatment, co-treatment or prevention of inflammation of the skin, in particular of sunburn in humans, of impure skin such as for example acne or of photoageing which is associated with chronic skin inflammation, said method comprising the step of administering an effective amount of the dermatological composition according to the invention to humans, which are in need thereof. Also, the invention relates to a method for cosmetic treatment, co-treatment or prevention of inflammation of the skin, in particular of sunburn or of impure skin by a cosmetic preparation according to the invention. Sunburn prevention is preferably achieved with topical application comprising the composition of the invention preferably in combination with suitable light screening agents.

The cosmetic or dermatological preparations according to the invention may be in the form of a suspension or dispersion in solvents or fatty substances, or alternatively in the form of an emulsion or micro emulsion (in particular of O/W or W/O type, O/W/O or W/O/W-type, wherein O stands for oil phase and wherein W stands for water phase), such as a cream, a paste, a lotion, a thickened lotion or a milk, a vesicular dispersion in the form of an ointment, a gel, a solid tube stick or an aerosol mousse, and may be provided in the form of a mousse, foam or a spray foams, sprays, sticks or aerosols or wipes. Examples of cosmetic or dermatological preparations are skin care preparations, in particular, body oils, body lotions, body gels, treatment creams, skin protection ointments, moisturizing gels, moisturizing sprays, revitalizing body sprays, after sun preparations or sunscreen formulations.

The cosmetic or dermatological composition for the treatment, co-treatment or prevention of inflammation of the skin, such as for example sunburn, photoageing or impure skin may be in a form that is conventional for oral administration, examples of which are described above and also include beauty foods and supplements.

The cosmetic or dermatological preparations of the invention for instance as sunscreen formulations or after sun preparations may further comprise the usual cosmetic respectively dermatological adjuvants and/or additives such as preservatives/ antioxidants, fatty substances/ oils, water, organic solvents, silicones, thickeners, softeners, emulsifiers, additional light screening agents, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, light stabilizers, insect repellants, skin tanning agents, skin whitening agents, antibacterial agents, preservatives active ingredients or any other ingredients usually formulated into cosmetics.

Light screening agents which may be incorporated into cosmetic or dermatological preparations of the invention for instance sunscreen formulations are advantageously selected from IR, UV-A, UV-B, UV-C and/ or broadband filters. Examples of UV-B or broad spectrum screening agents, *i*.*e*. substances having absorption maximums between about 290 and 340 nm may be organic or inorganic compounds. Organic UV-B or broadband screening agents are *e*.*g*. acrylates such as 2-ethylhexyl 2-cyano-3,3-diphenylacrylate (octocrylene, PARSOL^{®} 340), ethyl 2-cyano-3,3-diphenylacrylate and the like; camphor derivatives such as 4-methyl benzylidene camphor (PARSOL^{®} 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like; Cinnamate derivatives such as ethylhexyl methoxycinnamate (PARSOL^{®} MCX), ethoxyethyl methoxycinnamate, diethanolamine methoxycinnamate (PARSOL^{®} Hydro), isoamyl methoxycinnamate and the like as well as cinnamic acid derivatives bond to siloxanes; p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate; benzophenones such as benzophenone-3, benzophenone-4, 2,2', 4, 4'-tetrahydroxy-benzophenone, 2,2'-dihydroxy-4,4'-dimethoxybenzophenone and the like; esters of benzalmalonic acid such as di-(2-ethylhexyl) 4-methoxybenzalmalonate; esters of 2-(4-ethoxy-anilinomethylene)propandioic acid such as 2-(4-ethoxy anilinomethylene) propandioic acid diethyl ester as described in the European Patent Publication EP 0895 776; organosiloxane compounds containing benzmalonate groups as described in the European Patent Publications EP 0358584 B1, EP 0538431 B1 and EP 0709080 A1 such as polysilicone-15 (PARSOL^{®} SLX); drometrizole trisiloxane (Mexoryl XL); imidazole derivatives such as *e*.*g*. 2-phenyl benzimidazole sulfonic acid and its salts (PARSOL^{®}HS). Salts of 2-phenyl benzimidazole sulfonic acid are *e*.*g*. alkali salts such as sodium- or potassium salts, ammonium salts, morpholine salts, salts of primary, sec. and tert. amines like monoethanol amine salts, diethanol amine salts and the like; salicylate derivatives such as isopropylbenzyl salicylate, benzyl salicylate, butyl salicylate, ethylhexyl salicylate (PARSOL^{®} EHS, NEO Heliopan OS), isooctyl salicylate or homomenthyl salicylate (homosalate, PARSOL^{®} HMS, NEO Heliopan OS) and the like; triazine derivatives such as ethylhexyl triazone (Uvinul T-150), diethylhexyl butamido triazone (Uvasorb HEB). Encapsulated UV-filters such as encapsulated ethylhexyl methoxycinnamate (Eusolex UV-pearls) or microcapsules loaded with UV-filters as *e*.*g*. disclosed in EP 1471995 and the like. Inorganic compounds are pigments such as microparticulated TiO₂, ZnO and the like. The term "microparticulated" refers to a particle size from about 5 nm to about 200 nm, particularly from about 15 nm to about 100 nm. The TiO₂ particles may also be coated by metal oxides such as *e*.*g*. aluminum or zirconium oxides or by organic coatings such as *e*.*g*. polyols, methicone, aluminum stearate, alkyl silane. Such coatings are well known in the art.

Examples of broad spectrum or UV A screening agents *i*.*e*. substances having absorption maxima between about 320 and 400 nm may be organic or inorganic compounds *e*.*g*. dibenzoylmethane derivatives such as 4-tert. butyl-4'-methoxydibenzoyl-methane (PARSOL® 1789), dimethoxydibenzoylmethane, isopropyldibenzoylmethane and the like; benzotriazole derivatives such as 2,2'-methylene-bis-(6-(2H-benzotriazole-2-yl)-4-(1,1,3,3,-tetramethylbutyl)-phenol (TINOSORB M) and the like; bis-ethylhexyloxyphenol methoxyphenyl triazine (Tinosorb S) and the like; phenylene-1,4-bis-benzimidazolsulfonic acids or salts such as 2,2-(1,4-phenylene)bis-(1H-benzimidazol-4,6-disulfonic acid) (Neoheliopan AP); amino substituted hydroxybenzophenones such as 2-(4-Diethylamino-2-hydroxy-benzoyl)-benzoic acid hexylester (Uvinul A plus) as described in the European Patent Publication EP 1046391; Ionic UV-A filters as described in the International Patent Publication WO2005080341 A1. Pigments such as microparticulated ZnO or TiO2 and the like. The term "microparticulated" refers to a particle size from about 5 nm to about 200 nm, particularly from about 15 nm to about 100 nm. The particles may also be coated by other metal oxides such as *e*.*g*. aluminum or zirconium oxides or by organic coatings such as *e*.*g*. polyols, methicone, aluminum stearate, alkyl silane. Such coatings are well known in the art.

As dibenzoylmethane derivatives have limited photostability, it may be desirable to photostabilize these UV-A screening agents. Thus, the term "conventional UV-A screening agent" also refers to dibenzoylmethane derivatives such as *e*.*g*. PARSOL^{®} 1789 stabilized by, *e*.*g*. 3,3-Diphenylacrylate derivatives as described in the European Patent Publications EP 0 514 491 B1 and EP 0 780 119 A1; Benzylidene camphor derivatives as described in the US Patent No. 5,605,680; Organosiloxanes containing benzmalonate groups as described in the European Patent Publications EP 0358584 B1, EP 0538431 B1 and EP 0709080 A1.

Active ingredients which may be included in the cosmetic or dermatological preparations of the invention are for example vitamins and derivatives thereof, for example tocopherol, tocopherol acetate, ascorbic acid, ascorbyl phosphate, vitamin Q, D, and K, retinol, retinal, retinoic acid, retinol acetate, retinol palmitate, biotin, carotenoid derivatives such as beta-carotene, lycopene, asthaxanthin, vegetable extracts, antibacterial ingredients, instable amino acids comprising dipeptides, oligopeptides and polypeptides such as methionine, cysteine, cystine, tryptophan, phenylalanine, tyrosine, phenols, polyphenols or flavanoids, bisabolol, allantoin, phytantriol, panthenol, AHA acids, ubiquinones such as coenzyme Q 10, ceramides, pseudoceramides, essential oils, plant extracts deoxyribonucleic acid, phytanic acid.

The necessary amounts of the cosmetic and dermatological adjuvants, additives and/or additional active ingredients can, based on the desired product, easily be chosen by a person skilled in the art and will be illustrated in the examples, without being limited hereto.

In yet another embodiment, the invention relates to the use of magnolol and/ or honokiol for enhancing the anti-inflammatory activity of chondroitin.

It has been found that the compositions according to the invention are also suitable for the treatment, co-treatment or prevention of another joint disorder namely cartilage degradation or cartilage damage in joints and as such for treatment of the cartilage degradation component of joint disorders, for example degenerative joints disorders such as osteoarthritis; or sport injuries.

Cartilage degradation is defined within the framework of the invention as a metabolic disorder of joint cartilage characterized by increased production of cartilage-degrading enzymes such as matrix metalloproteases.

Osteoarthritis is a chronic degenerative disease of the joint of non-inflammatory origin, which develops by wear and tear of the joints during aging and results in pain and diminished joint function. Symptoms of osteoarthritis include pain, stiffness and loss of mobility in one or more joints. Excessive joint loading increases the risk of osteoarthritis, hence osteoarthritis mostly affects the weight-bearing joints such as spine, knees and hips, but thumb and finger joints may also be affected. Joint disorders can also results from injury, i.e. microdamage or blunt trauma, fractures, damage to tendons, menisci or ligaments or can be the result of excessive mechanical stress or other biomechanical instability resulting from for example an injury or obesity.

Joint disorders due to cartilage degradation are leading causes of disability and dysfunction in the elderly; almost 80% of people over age 60 show some evidence of these disorders. Age, genetic factors, muscle disuse and weakness, trauma, obesity and anatomical abnormalities contribute to the development of the disorder.

Joint disorders are difficult to treat. Up till now, treatment was largely limited to addressing the symptoms mainly with non-steroidal anti-inflammatory drugs. The drugs are given to control the pain and to restrain swelling, but do not prevent or treat damage to the cartilage. The patients experiencing severe cartilage damage frequently require surgery, including joint replacement surgery. Therefore, there was a great need for agents that treat or prevent cartilage loss and damage, which need has been solved by the present invention.
The compositions of the present invention may have one or more of the following properties:
it maintains and/or improves joint health, it prevents joint stiffness, it promotes joint mobility, it provides supple and/or flexible joints, it lubricates the joints, it relieves arthritis pain, it lessens joint problems, it provides joint care, it treats or prevents joint degradation, it provides joint integrity, it retards or prevents the progression of joint damage, it supports joint function, it promotes joint health and function, it naturally supports joint health and mobility for active individuals, it maintains the active flexibility of joints, it promotes joint flexibility and promotes joint mobility.
Thus, further objects of the present invention are:
- Use of a composition according to the invention as cartilage-regenerating and - maintaining agents.
- Use of a composition according to the invention (for the manufacture of a composition) for the maintenance and regeneration of articular cartilage.
- A method for the regeneration and/or maintenance of (articular) cartilage in a mammal which comprises administering to a mammal in need of such regeneration and/or maintenance an effective amount of a composition according to the invention.

The invention will now be elucidated by way of the following examples, without however being limited thereto.

### Brief description of the figures

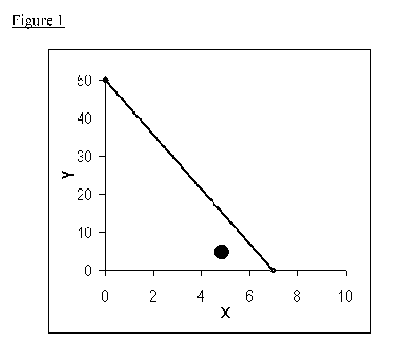

Figure 1: Synergistic effects of a mixture of MA/HO (1:3) and chondroitin sulfate on the inhibition of the inflammatory mediator nitric oxide (NO).
The x-axis indicates the concentration (in µmol/L) of MA/HO (1:3 w/w) and the y-axis indicates the concentrations (in mg/L) of chondroitin sulfate. The end points of the straight line reflect the IC₅₀ values of chondroitin sulfate (y-axis) and of MA/HO (1:3 w/w) (x-axis). The observed IC₅₀ value of the combination of chondroitin sulfate and MA/HO (1:3 w/w) is plotted by the circular symbol. It lies below the straight line and thus mirrors synergistic interactions.

Magnolia Bark extract is e.g. commercially available at Novanat Bioresources Co. Ltd. China. Honokiol (HO) and magnolol (MA) were from Honsea, Guangzhou, P.R. China and chondroitin sulfate (from shark) was purchased from Sigma.

### Example 1: Anti inflammatory activity of a combination of magnolol, honokiol and chondroitin sulfate

The anti-inflammatory effect of a mixture of MA/HO (1:3 w/w) in combination with chondroitin sulfate was determined in cellular assays by measuring the inhibition of the synthesis of nitric oxide. Nitric oxide (NO) is a hallmark of inflammation in various chronic inflammatory diseases including various forms of arthritis, gastro-intestinal diseases and metabolic syndrome X.
The effects on the inflammatory response were tested in cellular assays using a murine macrophage cell line, RAW264.7. The cells were purchased from ATCC (Manassas, VA, USA) and cultured in DMEM containing streptomycin/penicillin, non-essential amino acids and 10% fetal calf serum (FCS). In order to test a large range of concentration of a mixture of MA and HO (1:3) as well as mixtures thereof, cells (∼50'000/well) were seeded into flat-bottomed microtiter plates and cultured for one day. Cells were then starved in complete medium containing 0.25% FCS (D-025). After overnight culture, medium was removed and replaced by 100 µL of D-025 containing the test compounds at twice the final concentration. Subsequently, 100 µL of D-025 containing 2 µg/ml LPS was added (*i*.*e*. final LPS concentration of 1 µg/ml) and the cells cultured for 24 hours. Substances were usually tested in a concentration range from 0.2 to 50 µM in twofold dilution steps. All treatments were done in duplicates and several experimental series were done for each treatment. Concentrations of nitrite which was rapidly formed from nitric oxide released by cells were determined by the Griess reaction using sodium nitrite as standard. Briefly, 50 µl of supernatant was mixed with Griess reagent 1 (25 µL) and Griess reagent 2 (25 µL), centrifuged and the optical density at 540 nm determined. All determinations were done in duplicates and at various dilutions of the culture supernatant. IC₅₀ values for LPS-stimulated cells were calculated using a two-parametric least-square fitting equation [y = A+((B-A) / (1+((C-x)^D))] for best-fit curves (Excel fit software program).

As can bee seen in table 1 chondroitin sulfate only show a marginal anti-inflammatory whereas the mixture of MA/HO (1:3 w/w) already itself exhibits an anti-inflammatory activity.

**Table 1: IC₅₀ values for single substances**

| *Substance* | *IC₅₀Nitric Oxide* |
|---|---|
| Chondroitin sulfate | >50 mg/L |
| MA/HO (1:3 w/w) | 7.0 µmol/L |

The synergistic effect of a combination of a mixture of MA/HO (1:3 w/w) with chondroitin sulfate (ratio MA/HO (1:3 w/w) to chondroitin sulfate 13.3 mg/L (i.e. 50µmol/L) to 500 mg/L) on the inhibition of the inflammatory mediator nitric oxide (NO) has been evaluated based on the method published by Chou and Talalay (A simple generalized equation for the analysis of multiple inhibitions of Michealis-Menten kinetic systems. J. Biol. Chem. 1977. 252: 6438-6442). The results are visualized in an isobologram wherein synergistic interactions between substances are reflected by an experimental value that lies below the straight line (see also: Bitler et al., J Nutr 2005. 135: 1475-1479).
As can bee seen in Figures 1, the combination of magnolol, honokiol and chondroitin sulfate synergistically enhances the anti-inflammatory activity

### Example 2: Synergistic effect of the combination MA/ HO (1:3) with chondroitin sulfate on chondrocytes (cartilage build-up)

In articular cartilage, the balance between anabolic (build-up) and catabolic (break down) events needs to be maintained in order to prevent hypertrophy and excessive degradation of extracellular matrix (ECM), respectively. The ECM is built up of collagen and proteoglycans that are the products of collagen genes, for example human collagen 1 or aggrecan genes which are active during anabolic events. A substance which increases expression of these genes or the respective proteins (i.e. collagen and aggrecan) favours cartilage regeneration and/ort build-up.
Catabolic events are controlled by the expression of genes, *e*.*g*. those genes that encode matrix metalloproteinases (MMPs) that eventually break down collagen or proteoglycans (ADAMTS-4, - 5). Of the MMPs, MMP-1 and MMP-3 have a major role in breaking down the ECM in cartilage degradation. Some genes including TIMPS-1 (tissue-inhibitor of MMPs) have anti-catabolic effects and thus contribute to prevention of tissue erosion.
The effect of a MA/HO (1:3 w/w) mixture in combination with chondroitin was tested on the expression of human aggrecan, collagen I, collagen II, or chondrocyte transcription factors (SOX-6, -9) *in vitro* in normal human chondrocytes (derived from knee) (CC-2550; Cambrex, respectively). A control experiment without the compounds was done concomitantly to compare the expression of these genes (percentage expression of gene in controls was set to 100%). Normal human chondrocytes were cultured for 4 hours with MA/HO (1:3 w/w) (at 12.5 µmol/L), chondroitin (at 500 mg/L) as well as with both. The level of mRNA was determined by RT-PCR (Richard et al. Mol. Nutr. Food Res. 49, 431-442, 2005) and expressed relative to the level observed in cells cultured without the substances. The given values indicate the level of gene expression (in % of unstimulated cells [set at 100%] for anabolic and anti-catabolic genes; in % of IL1β-treated cells [set at 100%], for catabolic genes).

The mixture of MA/HO (1:3 w/w) was used at 12.5µmol/L (=3.325 mg/L), while the chondroitin sulfate (CS) was tested at 500 mg/L. The results shown in Table 2 indicate that a mixture of MA/ HO (1:3 w/w) substantially increases the expression of anabolic genes such as aggrecan. Similar effects were elicited by chondroitin. Yet, when combined the observed increase in gene expression exceeded the expected value reflecting a synergistic interaction. Similar features were observed for the anti-catabolic gene TIMPS-1. Conversely, the expression of MMP-2 and ADAMTS-5 was synergistically reduced.

**Table 2**

| *Type ofgene* | *Gene* | *MA*/*HO (1:3)* | *CS* | *MA*/*HO* + *CS* |
|---|---|---|---|---|
| | | *(3.325 mg*/*L)* | *(500 mg*/*L)* | *(3.325 mg*/*L + 500 mg*/*L)* |
| Anabolic | Aggrecan (stim., 4hrs) | 147 | 62 | **173** ¹⁾ |
| Anabolic | SOX-9 | 87 | 147 | **152** ¹⁾ |
| Anti-catabolic | TIMP-1 (unstim., 4d) | 173 | 106 | **220** ¹⁾ |
| Catabolic | MMP-2 (4d) | 93 | 93 | **70**²⁾ |
| Catabolic | ADAMTS-5 (stim. 4hrs) | 100 | 107 | **75**²⁾ |

| | | | | |
|---|---|---|---|---|
| ¹⁾ For anabolic and anti-catabolic genes an increased expression level (compared to control which is set at 100%) reflects a cartilage-rebuilding activity. ²⁾ For catabolic genes a decreased expression level reflects a chondroprotective effect. | | | | |

## Claims

1. A composition comprising magnolol and/ or honokiol and chondroitin.

2. The composition as in claim 1, wherein the magnolol and/ or honokiol is in the form of an extract from the bark of *Magnolia officinalis* comprising magnolol and/ or honokiol.

3. The composition as in claim 1 or 2, wherein the ratio of magnolol and / or honokiol to chondroitin is in the range of 1 to 10 to 1 to 1.

4. A use of a composition as in any one of claims 1 to 3 as an agent for the treatment, co-treatment or prevention of inflammatory disorders.

5. A use of a composition as in any one of claims 1 to 3 as an agent for treatment, co-treatment and prevention of joint disorders.

6. A nutraceutical comprising a composition as defined in any of claims 1 to 3 and a nutraceutically acceptable carrier.

7. The nutraceutical as in claim 6 which is a food product, foodstuff, dietary supplement, nutritional supplement or a supplement composition for a food product or a foodstuff.

8. The nutraceutical composition as in claim 6 and 7 wherein the amount of magnolol and/ or honokiol is 0.01 to 1g, more preferably 0.2 mg to 500 mg per serving.

9. The composition as in any one of claims 1 to 3 for use as a medicament.

10. A use of a composition as defined in any one of claims 1 to 3, for the manufacture of a medicament for the treatment, co-treatment or prevention of inflammatory disorders.

11. The use as in claim 10, wherein the inflammatory disorder is arthritis.

12. The use as in claim 10 wherein the inflammatory disorder is an inflammation of the skin.

13. A pharmaceutical comprising a composition as defined in any of claims 1 to 3 and a pharmaceutically acceptable carrier.

14. The pharmaceutical as in claim 13, which is in the form of a powder, tablet, capsule, gel, liquid or solid.

15. The pharmaceutical as in claim 13, which is for dermatological purposes.

16. A cosmetic composition comprising a composition as defined in any one of claims 1 to 3 and a cosmetically acceptable carrier.

17. The cosmetic composition as in claim 16 which is a skin care preparation.

18. A method for treatment, co-treatment or prevention of inflammatory disorders in animals said method comprising the step of administering an effective amount of the composition as defined in any one of claims 1 to 3 to an animal, which are in need of such a treatment.

19. The method as in claim 18, wherein the inflammatory disorder is arthritis.

20. The method as in claim 18, wherein the inflammatory disorder is an inflammation of the skin.

21. Use of magnolol and/ or honokiol for enhancing the anti-inflammatory activity of chondroitin.

22. Method of enhancing the efficacy of magnolol and/ or honokiol which comprises adding to a composition containing magnolol and/ or honokiol an effective amount of chondroitin
